# NEUE EUROPÄISCHE PATENTSCHRIFT

(11) **EP 3 030 214 B2**
(45) Veröffentlichungstag und Bekanntmachung des Hinweises auf die Entscheidung über den Einspruch: **17.01.2024**
(45) Hinweis auf die Patenterteilung: 24.02.2021
(21) Anmeldenummer: 14730832.4
(22) Anmeldetag: 11.06.2014
(51) Int. Cl.: A61K 8/04, A61K 8/34, A61K 8/73, A61Q 17/04

(54) **GELFÖRMIGES, ALKOHOLISCHES SONNENSCHUTZMITTEL**
ALCOHOLIC SUNSCREEN GEL
PRODUIT DE PROTECTION SOLAIRE SOUS FORME DE GEL À TENEUR EN ALCOOL

(30) Priorität: 09.08.2013 DE 102013215831
(43) Veröffentlichungstag der Anmeldung: 15.06.2016
(73) Patentinhaber: Beiersdorf AG, 20253 Hamburg (DE)
(72) Erfinder: BORCHERS, Kathrin, 21614 Buxtehude (DE); TESCH, Mirko, 22303 Hamburg (DE); BLECKMANN, Andreas, 22926 Ahrensburg (DE); MEYER, Christiane, 20357 Hamburg (DE)
(74) Vertreter: Beiersdorf AG
(86) Internationale Anmeldenummer: PCT/EP2014/062090
(87) Internationale Veröffentlichungsnummer: WO 2015/018549

(56) Entgegenhaltungen:
- EP-A1- 0 812 586
- WO-A1-2007/068699
- WO-A1-2013/172990
- WO-A2-2007/140442
- WO-A2-2007/140442
- WO-A2-2009/124632
- US-A- 5 017 366
- US-A1- 2006 073 106
- US-A1- 2006 073 106
- US-A1- 2008 253 978
- US-A1- 2008 305 133
- US-A1- 2011 250 155
- Ashland: "Klucel RTM hydroxypropylcellulose - physical and chemical properties", , 1. Januar 2012 (2012-01-01), XP055142638, Gefunden im Internet: URL:http://www.ashland.com/Ashland/Static/ Documents/ASI/PC_11229_Klucel_HPC.pdf [gefunden am 2014-09-25]
- Auszug aus der Datenbank Mintel Record-ID 737033

## Beschreibung

Die vorliegende Erfindung betrifft eine gelförmige kosmetische Zubereitung enthaltend Ethanol, ein oder mehrere UV-Filter und Hydroxypropylcellulose mit einem Molekulargewicht von kleiner als 1000 kg/mol, sowie deren Verfahren zur Herstellung und die Verwendung der Hydroxypropylcellulose in der Zubereitung.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB-und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine in jüngerer Zeit populär gewordene Produktform von Sonnenschutzmitteln stellen die transparenten Gele dar. Diese werden in der Regel auf Wasser- oder Alkoholbasis formuliert und mit Zellulosederivaten (z.B. Hydroxyethyl- oder Hydroxypropylderivaten), Acrylaten (z.B. Acrylates/C10-30 Alkylacrylates) oder PVP auf eine entsprechende Viskosität hin verdickt. Nachteilig am Stande der Technik ist jedoch der Umstand, dass sich (nach herrschender Meinung) der Lichtschutzfaktor SPF dieser Zubereitungen allein durch den Gehalt an UV-Filtern einstellen lässt. Die für die Kosmetik zugelassenen UV-Filter sind jedoch in der Regel, aufgrund ihres komplizierten Molekülaufbaus und der damit einhergehenden komplexen Synthese, relativ teuer und häufig schwer in größeren Konzentrationen stabil in Lösung zu halten. Darüber hinaus führt ein hoher Gehalt an UV-Filtern in den Zubereitungen dazu, dass diese sensorisch unattraktiv werden, wobei hier insbesondere die Zunahme an der Klebrigkeit der Rezeptur und der Neigung zur "Röllchenbildung" beim Verreiben auf der Haut zu nennen ist.

Es war daher die Aufgabe der vorliegenden Erfindung gewesen, sensorisch attraktive transparente kosmetische Sonnenschutzgele zu entwickeln und Mittel und Wege zu finden um den Lichtschutzfaktor (SPF) von transparenten Sonnenschutzgelen zu erhöhen ohne die die UV-Filterkonzentration zu verändern (d.h. weiter zu erhöhen).

Überraschend gelöst wird die Aufgabe durch eine gelförmige kosmetische Zubereitung gemäß der Ansprüche 1 und 2.

Dabei bedeutet "definierte Viskosität", dass die Viskosität der Zubereitung vorgegeben ist und die Steigerung des Lichtschutzfaktors im Vergleich zu einer Zubereitung gleicher Viskosität auftritt, die mit einem anderen, nicht erfindungsgemäßen Verdickungsmittel verdickt wurde.

Zwar kennt der Stand der Technik die EP2066286 und EP 2288416, doch konnten diese Schriften nicht den Weg zur vorliegenden Erfindung weisen. Ferner kennt der Fachmann die US 5017366A, US 2008/253978 A1, WO 2013/172990 A1, US 2011/250155 A1, WO 2007/140442 A2, US 2006/073106 A1 und Ashland: "Klucel RTM hydroxypropylcellulosephysical and chemical properties" vom 1. Januar 2012, (XP055142638, die ebenfalls nicht den Weg zur vorliegenden Erfindung weisen konnten.

Es ist erfindungsgemäß vorteilhaft, wenn die erfindungsgemäße Zubereitung Ethanol in einer Menge von 25 bis 75 Gewichts-%, bezogen auf das Gesamtgewicht der Zubereitung, enthält. Erfindungsgemäß bevorzugt ist dabei ein Ethanolgehalt von 45 bis 65 Gewichts-%, bezogen auf das Gesamtgewicht der Zubereitung.

Erfindungsgemäße Ausführungsformen der vorliegenden Erfindung sind dadurch gekennzeichnet, dass die Zubereitung Hydroxypropylcellulose mit einem Molekulargewicht Mw von kleiner als 1000 kg/mol gemessen mittels Größenausschlusschromatographie beinhalten.

Es ist dabei erfindungsgemäß bevorzugt, wenn das Molekulargewicht Mw der Hydroxypropylcellulose zwischen 800 und 900 kg/mol liegt (gemessen mittels Größenausschlusschromatographie).

Es ist vorteilhaft im Sinne der vorliegenden Erfindung die Hydroxypropylcellulose in einer Menge von 0,3 bis 3,0 Gewichts-%, bezogen auf das Gesamtgewicht der Zubereitung, einzusetzen.

Dabei ist ein Gehalt von 1,0 bis 1,5 Gewichts-%, bezogen auf das Gesamtgewicht der Zubereitung an Hydroxypropylcellulose mit einem Molekulargewicht Mw von kleiner als 1000 kg/mol, gemessen mittels Größenausschlusschromatographie, erfindungsgemäß bevorzugt. Eine erfindungsgemäß vorteilhafte Hydroxypropylcellulose kann beispielsweise unter dem Handelsnamen Klucel MF erworben werden.

Vorteilhafte Ausführungsformen der erfindungsgemäßen Zubereitung, deren Verwendung und eines demgemäßen Verfahrens sind dadurch gekennzeichnet, dass die Zubereitung eine Viskosität von 1200 bis 3000mPas aufweist. Die Messung erfolgte bei 25°C mithilfe eines Kegel-Platte Viskosimeter mit einem Durchmesser von 40mm und mit einer Scherrate von 10s-1.

Es ist erfindungsgemäß besonders vorteilhaft, wenn die erfindungsgemäße Zubereitung transparent ist.

Dabei gilt eine Zubereitung erfindungsgemäß und anspruchsgemäß als transparent, wenn es möglich ist, bei Tageslicht durch eine mit der erfindungsgemäßen Zubereitung gefüllten Einmal-Küvette (Firma Brand, 2,5ml, Wellenlängenbereich: 220nm-900nm) mit dem bloßen Auge zu schauen. Schriftzeichen (Schrifttyp Arial Schriftgröße 10), die sich unmittelbar hinter der Einmal-Küvette befinden, sollten erkennbar und lesbar sein.

Es ist erfindungsgemäß vorteilhaft, wenn die erfindungsgemäße Zubereitung einen oder mehrere UV-Filter gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; ,4-Bis-{[4-(2-ethyl-hexyloxy)-2-hydroxy]-phenyl}-6-(4-methoxyphenyl)-1,3,5-triazin; 3-Benzylidencampher; Ethylhexylsalicylat; Terephthalidendicamphersulfonsäure; 4-(tert.-Butyl)-4'-methoxydiben zoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethyl-hexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tribiphenyl-4-yl-1,3,5-triazin; Merocyanine; Titandioxid; Zinkoxid, enthält.

Die erfindungsgemäß bevorzugten UV-Filter sind dabei: 4-Bis-{[4-(2-ethyl-hexyloxy)-2-hydro-xy]-phenyl}-6-(4-methoxyphenyl)-1,3,5-triazin; Ethylhexylsalicylat; 4-(tert.-Butyl)-4'-methoxy-dibenzoylmethan; 2-Ethylhexyl-2-cyano-3,3-diphenylacrylat; 4-Methoxyzimtsäure(2-ethylhexyl)ester; Homomenthylsalicylat; Dioctylbutylamidotriazon (INCI: Diethylhexyl-Butamidotriazone); 3-(4-(2,2-bis Ethoxycarbonylvinyl)-phenoxy)propenyl)-methoxysiloxan / Dimethylsiloxan - Copolymer

Es ist erfindungsgemäß vorteilhaft, wenn die erfindungsgemäße Zubereitung UV-Filter in einer Konzentration von 6 bis 35 Gewichts-%, bezogen auf das Gesamtgewicht der Zusammensetzung enthält, wobei ein Gehalt von 25 bis 35 Gewichts-%, bezogen auf das Gesamtgewicht der Zubereitung, erfindungsgemäß bevorzugt ist.

Erfindungsgemäß vorteilhafte Ausführungsformen der vorliegenden Erfindung sind dadurch gekennzeichnet, dass der Wassergehalt der Zubereitung kleiner oder gleich 3,0 Gewichts-%, bezogen auf das Gesamtgewicht der Zubereitung, beträgt.

Erfindungsgemäß vorteilhafte Ausführungsformen der vorliegenden Erfindung sind dadurch gekennzeichnet, dass die Gesamtmenge (Summe) an Cetylalkohol, Cetearylalkohol und Myristylalkohol in der Zubereitung von 0,5 bis 7,5 Gewichts-%, bezogen auf das Gesamtgewicht der Zubereitung, beträgt. Dabei hat es für diese Gesamtmenge keine Bedeutung, ob ein, zwei oder alle drei Alkohole enthaltend sind. Sie ist in allen Fällen die gleiche.

Erfindungsgemäß bevorzugt ist es die Gesamtmenge (Summe) dieser Fettalkohole in einer Konzentration von 2,5 bis 5,0 Gewichts-%, bezogen auf das Gesamtgewicht der Zubereitung einzusetzen.

Erfindungsgemäß vorteilhafte Ausführungsformen der vorliegenden Erfindung sind dadurch gekennzeichnet, dass die Gesamtmenge (Summe) an Isoprpopylstearat, Dicaprylylether, Isopropylpalmitat und Ethylhexylstearat in der Zubereitung von 0,5 bis 2,0 Gewichts-%, bezogen auf das Gesamtgewicht der Zubereitung, beträgt. Dabei hat es für diese Gesamtmenge keine Bedeutung, ob ein, zwei oder alle drei Verbindungen enthaltend sind. Sie ist in allen Fällen die gleiche.

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

Darüber hinaus kann die erfindungsgemäße Zubereitung vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl- oder-monobutylether, Propylenglykolmonomethyl, -monoethyl- oder -monobutylether, Diethylenglykolmonomethyl-oder -monoethylether und analoge Produkte.

Erfindungsgemäß vorteilhaft enthält die erfindungsgemäße Zubereitung ein oder mehrere Parfümstoffe.

Erfindungsgemäß bevorzugt enthält die erfindungsgemäße Zubereitung ein oder mehreren Parfümstoffen gewählt aus der Gruppe 2-lsobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin, Limonen [5989-27-5], Citral, Linalool [78-70-6], alpha-Isomethylionon [1335-46-2], Geraniol [106-24-1], Citronellol [106-22-9], [24851-98-7], [18479-58-8], [54464-57-2], [80-54-6], [1222-05,5], [32388-55-9], [105-95-3], [31906-04-4], [8008-57-9], [32210-23-4], [120-57-0], [115-95-7], [101-86-0], [140-11-4], [6259-76-3] und [127-51-5].

In einer erfindungsgemäß vorteilhaften Ausführungsform ist die erfindungsgemäße Zubereitung frei von C12-13 Pareth-9.

Die erfindungsgemäßen Zubereitungen können beispielsweise als Sonnenschutzmittel oder Tagespflegeprodukt verwendet werden.

Die erfindungsgemäßen Zubereitungen können auch als Spray oder Tränkung eingesetzt werden.

### Vergleichsversuche

In den Formulierungen wurden verschiedene Hydroxypropylcellulose-Typen der Firma Ashland eingesetzt. Es wurden vergleichende SPF-Screenings mit den unterschiedlichen KLUCEL-Typen durchgeführt. Dabei stellte sich heraus (siehe Zeichnung), dass der verwendete KLUCEL-Typ einen Einfluss auf das SPF-Ergebnis hat. In dem nachfolgenden Diagramm sehen Sie die SPF-Screening-Ergebnisse von vergleichbaren Formeln mit KLUCEL HXF und KLUCEL MF. Die Einsatzkonzentraion der KLUCEL-Typen ist aufgrund der verschiedenen Verdickungsleistung und der festgelegten Zielviskosität von 3000 mPas unterschiedlich gewählt.

| Handelsname | #1 | #2 | #3 | #4 |
|---|---|---|---|---|
| **KLUCEL HXF** | **1,13** | - | **1,0** | - |
| **KLUCEL MF** | - | **1,5** | - | **1,5** |
| **Dermacryl 79** | 2,0 | 2,0 | 1,0 | 1,0 |
| **Sylvaclear A200V** | | | 3,0 | 3,0 |
| **Lanette 16** | 5,0 | 5,0 | 3,0 | 3,0 |
| **Viskosität 7d [mPas]** | 2850 | 3000 | 2100 | 3000 |
| **Valide Personen** | 6 von 6 | 6 von 6 | 5 von 6 | 6 von 6 |
| **SPF** | **22,5** | **30,3** | **24,2** | **30,1** |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Rohstoff-INCI** | **m [%]** | **m [%]** | **m [%]** |
|---|---|---|---|
| Butyl Methoxydibenzoylmethane | 4,50 | 4,50 | 4,75 |
| Octocrylene | 7,50 | 9,50 | 9,50 |
| Homosalate | 9,50 | 9,50 | 9,50 |
| Ethylhexyl Salicylate | 4,75 | 4,75 | 4,75 |
| Bis-Ethylhexyloxyphenol Methoxyphenyl Triazine | 0,60 | 2,25 | 2,25 |
| Polysilicone-15 | | 1,00 | 1,00 |
| Diethylhexyl Butamido Triazone | | 1,00 | 1,00 |
| Ethylhexyl Methoxycinnamate + BHT | | 0,50 | 0,50 |
| C12-15 Alkyl Benzoate | | 9,50 | 7,50 |
| Acrylates/Octylacrylamide Copolymer | 2,00 | 2,00 | 2,00 |
| Cetyl Alcohol | 4,00 | 5,00 | 5,00 |
| Hydroxypropylcellulose | 1,30 | 1,30 | 1,30 |
| Isopropyl Stearate | 1,00 | | |
| Glycerin | 1,00 | 1,00 | 1,00 |
| Parfum | 0,80 | 0,80 | 0,80 |
| Menthol | 0,01 | 0,01 | 0,01 |
| Alcohol Denat. | 63,04 | 47,39 | 49,14 |

## Patentansprüche

1. Gelförmige kosmetische Zubereitung enthaltend
a) Ethanol,
b) ein oder mehrere UV-Filter,
c) Hydroxypropylcellulose mit einem Molekulargewicht von kleiner als 1000 kg/mol, gemessen mittels Größenausschlusschromatographie,
wobei die Zubereitung frei ist von 3-(4-Methylbenzyliden)-campher und 2-Hydroxy-4-methoxybenzophenon (Oxybenzon), **dadurch gekennzeichnet, dass** die Zubereitung Cetylalkohol, Cetearylalkohol und/oder Myristylalkohol enthält.

2. Gelförmige kosmetische Zubereitung enthaltend
a) Ethanol,
b) ein oder mehrere UV-Filter,
c) Hydroxypropylcellulose mit einem Molekulargewicht von kleiner als 1000 kg/mol, gemessen mittels Größenausschlusschromatographie,
wobei die Zubereitung frei ist von 3-(4-Methylbenzyliden)-campher und 2-Hydroxy-4-methoxybenzophenon (Oxybenzon), **dadurch gekennzeichnet, dass** die Zubereitung Isoprpopylstearat, Dicaprylylether, Isopropylpalmitat und/oder Ethylhexylstearat enthält.

3. Zubereitung nach Anspruch 1 oder 2, **dadurch gekennzeichnet, dass** die Zubereitung Ethanol in einer Menge von 25 bis 75 Gewichts-%, bezogen auf das Gesamtgewicht der Zubereitung, enthält.

4. Zubereitung nach einem der vorhergehenden Ansprüche, **dadurch gekennzeichnet, dass** die Zubereitung Hydroxypropylcellulose in einer Menge von 0,3 bis 3,0 Gewichts-%, bezogen auf das Gesamtgewicht der Zubereitung enthält.

5. Zubereitung nach einem der vorhergehenden Ansprüche, **dadurch gekennzeichnet, dass** die Zubereitung eine Viskosität von 1200 bis 3000 mPas (gemessen bei 25°C mithilfe eines Kegel-Platte Viskosimeter mit einem Durchmesser von 40mm und mit einer Scherrate von 10s⁻¹ aufweist.

6. Zubereitung nach einem der vorhergehenden Ansprüche, **dadurch gekennzeichnet, dass** die Zubereitung transparent ist.

7. Zubereitung nach einem der vorhergehenden Ansprüche, **dadurch gekennzeichnet, dass** die Zubereitung einen oder mehrere UV-Filter gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; ,4-Bis-{[4-(2-ethyl-hexyloxy)-2-hydroxy]-phenyl}-6-(4-methoxyphenyl)-1,3,5-triazin; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalonsäuredi(2-ethylhexyl)ester; 4-Methoxyzimtsäure(2-ethylhexyl)ester; 4-Methoxyzimtsäureisoamylester; 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tribiphenyl-4-yl-1,3,5-triazin; Merocyanine; Titandioxid; Zinkoxid, enthält.

8. Zubereitung nach einem der vorhergehenden Ansprüche, **dadurch gekennzeichnet, dass** der Wassergehalt der Zubereitung kleiner oder gleich 3,0 Gewichts-%, bezogen auf das Gesamtgewicht der Zubereitung, beträgt.

9. Zubereitung nach Anspruch einem der vorhergehenden Ansprüche, **dadurch gekennzeichnet, dass** die Gesamtmenge (Summe) an Cetylalkohol, Cetearylalkohol und Myristylalkohol in der Zubereitung von 0,5 bis 7,5 Gewichts-%, bezogen auf das Gesamtgewicht der Zubereitung, beträgt.

10. Zubereitung nach einem der vorhergehenden Ansprüche, **dadurch gekennzeichnet, dass** die Gesamtmenge (Summe) an Isopropylstearat, Dicaprylylether, Isopropylpalmitat und Ethylhexylstearat in der Zubereitung von 0,5 bis 2,0 Gewichts-%, bezogen auf das Gesamtgewicht der Zubereitung, beträgt.

## Claims

1. Cosmetic gel preparation comprising
a) ethanol
b) one or more UV filters,
c) hydroxypropyl cellulose having a molecular weight of less than 1000 kg/mol, measured by size exclusion chromatography,
wherein the preparation is free from 3-(4-methylbenzylidene)camphor and 2-hydroxy-4-methoxybenzophenone (oxybenzone), **characterized in that** the preparation comprises cetyl alcohol, cetearyl alcohol and/or myristyl alcohol.

2. Cosmetic gel preparation comprising
a) ethanol
b) one or more UV filters,
c) hydroxypropyl cellulose having a molecular weight of less than 1000 kg/mol, measured by size exclusion chromatography,
wherein the preparation is free from 3-(4-methylbenzylidene)camphor and 2-hydroxy-4-methoxybenzophenone (oxybenzone), **characterized in that** the preparation comprises isopropyl stearate, dicaprylyl ether, isopropyl palmitate and/or ethylhexyl stearate.

3. Preparation according to Claim 1 or 2, **characterized in that** the preparation comprises ethanol in an amount of 25 to 75% by weight, based on the total weight of the preparation.

4. Preparation according to either of the preceding claims, **characterized in that** the preparation comprises hydroxypropyl cellulose in an amount of 0.3 to 3.0% by weight, based on the total weight of the preparation.

5. Preparation according to any of the preceding claims, **characterized in that** the preparation has a viscosity of 1200 to 3000 mPas (measured at 25°C using a cone-plate viscometer having a diameter of 40 mm and at a shear rate of 10 s⁻¹).

6. Preparation according to any of the preceding claims, **characterized in that** the preparation is transparent.

7. Preparation according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; ,4-bis-{[4-(2-ethylhexyloxy)-2-hydroxy]phenyl}-6-(4-methoxyphenyl)-1,3,5-triazine; 3-benzylidenecamphor; ethylhexyl salicylate; terephthalidenedicamphorsulfonic acid; 4-(tert-butyl)-4'-methoxydibenzoylmethane; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tribiphenyl-4-yl-1,3,5-triazine; merocyanine; titanium dioxide; zinc oxide.

8. Preparation according to any of the preceding claims, **characterized in that** the water content of the preparation is less than or equal to 3.0% by weight, based on the total weight of the preparation.

9. Preparation according to any of the preceding claims, **characterized in that** the total amount (sum) of cetyl alcohol, cetearyl alcohol and myristyl alcohol in the preparation is from 0.5 to 7.5% by weight, based on the total weight of the preparation.

10. Preparation according to any of the preceding claims, **characterized in that** the total amount (sum) of isopropyl stearate, dicaprylyl ether, isopropyl palmitate and ethylhexyl stearate in the preparation is from 0.5 to 2.0% by weight, based on the total weight of the preparation.

## Revendications

1. Préparation cosmétique sous forme de gel contenant
a) de l'éthanol,
b) un ou plusieurs filtres UV,
c) une hydroxypropylcellulose dotée d'un poids moléculaire inférieur à 1 000 kg/mole, mesuré au moyen d'une chromatographie d'exclusion stérique,
la préparation étant exempte de 3-(4-méthylbenzylidène)-camphre et de 2-hydroxy-4-méthoxybenzophénone (oxybenzone), **caractérisée en ce que** la préparation contient de l'alcool cétylique, de l'alcool cétéarylique et/ou de l'alcool myristylique.

2. Préparation cosmétique sous forme de gel contenant
a) de l'éthanol,
b) un ou plusieurs filtres UV,
c) une hydroxypropylcellulose dotée d'un poids moléculaire inférieur à 1 000 kg/mole, mesuré au moyen d'une chromatographie d'exclusion stérique,
la préparation étant exempte de 3-(4-méthylbenzylidène)-camphre et de 2-hydroxy-4-méthoxybenzophénone (oxybenzone), **caractérisée en ce que** la préparation contient du stéarate d'isopropyle, de l'éther de dicaprylyle, du palmitate d'isopropyle et/ou du stéarate d'éthylhexyle.

3. Préparation selon la revendication 1 ou 2, **caractérisée en ce que** la préparation contient de l'éthanol en une quantité de 25 à 75 % en poids, par rapport au poids total de la préparation.

4. Préparation selon l'une quelconque des revendications précédentes, **caractérisée en ce que** la préparation contient une hydroxypropylcellulose en une quantité de 0,3 à 3,0 % en poids, par rapport au poids total de la préparation.

5. Préparation selon l'une quelconque des revendications précédentes, **caractérisée en ce que** la préparation présente une viscosité de 1 200 à 3 000 mPas (mesurée à 25 °C à l'aide d'un viscosimètre à cône et plaque comportant un diamètre de 40 mm et d'une vitesse de cisaillement de 10 s⁻¹.

6. Préparation selon l'une quelconque des revendications précédentes, **caractérisée en ce que** la préparation est transparente.

7. Préparation selon l'une quelconque des revendications précédentes, **caractérisée en ce que** la préparation contient un ou plusieurs filtres UV choisis dans le groupe des composés acide 2-phénylbenzimidazole-5-sulfonique et/ou ses sels ; sels de l'acide phénylène-1,4-bis(2-benzymidazyl)-3,3'-5,5'-tétrasulfonique ; 1,4-di(2-oxo-10-sulfo-3-bornylidèneméthyl)benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2*H-*benzotriazol-2-yl)-4-(1,1,3,3-tétraméthylbutyl)phénol ; 2-(2*H*-benzotriazol-2-yl)-4-méthyl-6-[2-méthyl-3-[1,3,3,3-tétraméthyl-1-[(triméthylsilyl)oxy]disiloxanyl]propyl]phénol ; ,4-bis-{[4-(2-éthylhexyloxy)-2-hydroxy]-phényl}-6-(4-méthoxyphényl)-1,3,5-triazine ; 3-benzylidènecamphre ; salicylate d'éthylhexyle ; acide téréphtalidènedicamphresulfonique ; 4-(tert-butyl)-4'-méthoxydibenzoylméthane ; 2-cyano-3,3-diphénylacrylate de 2-éthylhexyle ; 4-diméthylaminobenzoate de 2-éthylhexyle ; 4-diméthylaminobenzoate d'amyle ; 4-méthoxybenzalmalonate de di(2-éthylhexyle) ; 4-méthoxycinnamate de 2-éthylhexyle ; 4-méthoxycinnamate d'isoamyle ; 2-hydroxy-4-méthoxy-4'-méthylbenzophénone ; 2,2'-dihydroxy-4-méthoxybenzophénone ; 2-(4'-diéthylamino-2'-hydroxybenzoyl)benzoate d'hexyle ; salicylate d'homomenthyle ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (numéro CAS 288254-16-0) ; 4,4',4"-(1,3,5-triazin-2,4,6-triyltriimino)-tris-benzoate de tris(2-éthylhexyle) (aussi : 2,4,6-tris-[anilino-(p-carbo-2'-éthyl-1'-hexyloxy)]-1,3,5-triazine (INCI : Ethylhexyl Triazone) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 2,4,6-tribiphényl-4-yl-1,3,5-triazine ; mérocyanine ; dioxyde de titane ; oxyde de zinc.

8. Préparation selon l'une quelconque des revendications précédentes, **caractérisée en ce que** la teneur en eau de la préparation est inférieure ou égale à 3,0 % en poids, par rapport au poids total de la préparation.

9. Préparation selon l'une quelconque des revendications précédentes, **caractérisée en ce que** la quantité totale (somme) d'alcool cétylique, d'alcool cétéarylique et d'alcool myristylique dans la préparation est de 0,5 à 7,5 % en poids, par rapport au poids total de la préparation.

10. Préparation selon l'une quelconque des revendications précédentes, **caractérisée en ce que** la quantité totale (somme) de stéarate d'isopropyle, d'éther de dicaprylyle, de palmitate d'isopropyle et/ou de stéarate d'éthylhexyle dans la préparation est de 0,5 à 2,0 % en poids, par rapport au poids total de la préparation.
